# EUROPEAN PATENT SPECIFICATION

(11) **EP 3 939 615 B1**
(45) Date of publication and mention of the grant of the patent: **05.06.2024**
(21) Application number: 20769713.7
(22) Date of filing: 27.04.2020
(51) Int. Cl.: A61K 47/36, A61K 47/18, A61K 8/41, A61K 8/60, A61Q 1/00, A61K 9/00, A61K 9/127, A61K 9/51, A61K 47/34

(54) **PREPARATION METHOD AND USE FOR ARTIFICIAL EXOCRINE COMPLEX**
HERSTELLUNGSVERFAHREN UND VERWENDUNG FÜR EINEN KÜNSTLICHEN EXOKRINEN KOMPLEX
PROCÉDÉ DE PRÉPARATION ET UTILISATION D'UN COMPLEXE EXOCRINE ARTIFICIEL

(30) Priority: 12.03.2019 CN 201910183081
(43) Date of publication of application: 19.01.2022
(73) Proprietor: Shanghai Cheermore Biological Technology Co., Ltd., Shanghai 201400 (CN)
(72) Inventor: ZHU, Caibin, Shanghai 201400 (CN); LI, Junxiang, Shanghai 201400 (CN)
(74) Representative: Bayramoglu et al.
(86) International application number: PCT/CN2020/087344
(87) International publication number: WO 2020/182226

(56) References cited:
- WO-A1-2016/144766
- CN-A- 101 821 317
- CN-A- 102 408 498
- CN-A- 102 408 498
- CN-A- 102 408 498
- CN-A- 103 665 384
- CN-A- 110 638 690
- CN-A- 110 638 690
- US-A1- 2010 197 888
- US-A1- 2012 202 283
- LUTZ TAUHARDT ET AL: MACROMOLECULAR CHEMISTRY AND PHYSICS, vol. 212, no. 17, 30 June 2011 (2011-06-30), pages 1918-1924, XP055732939, DE ISSN: 1022-1352, DOI: 10.1002/macp.201100190
- KITAE PARK ET AL: "Reducible Hyaluronic Acid-siRNA Conjugate for Target Specific Gene Silencing", BIOCONJUGATE CHEMISTRY, vol. 24, no. 7, 3 June 2013 (2013-06-03), pages 1201-1209, XP055732941, US ISSN: 1043-1802, DOI: 10.1021/bc4001257
- SAIBOM PARK ET AL: "Dual roles of hyaluronic acids in multilayer films capturing nanocarriers for drug-eluting coatings", BIOMATERIALS, ELSEVIER, AMSTERDAM, NL, vol. 33, no. 21, 26 April 2012 (2012-04-26), pages 5468-5477, XP028487156, ISSN: 0142-9612, DOI: 10.1016/J.BIOMATERIALS.2012.04.005
- SUNITI MISRA ET AL: "Interactions between Hyaluronan and Its Receptors (CD44, RHAMM) Regulate the Activities of Inflammation and Cancer", FRONTIERS IN IMMUNOLOGY, vol. 6, 6 May 2015 (2015-05-06), pages 1-31, XP055635727, DOI: 10.3389/fimmu.2015.00201
- Lutz Tauhardt , Kristian Kempe , Katrin Knop , Esra Altunta , Michael Jäger , Stephanie Schubert , Dagmar Fischer ,Ulrich S. Sch: "Linear Polyethyleneimine: Optimized Synthesis and Characterization-On the Way to Pharmagrade Batches", Macromolecular Chemistry and Physics, vol. 212, no. 17, 30 June 2011 (2011-06-30), pages 1918-1924, XP055732939, DOI: 10.1002/macp.201100190
- Kitae Park, Jeong-A Yang, Min-Young Lee, Hwiwon Lee,Sei Kwang Hahn: "Reducible Hyaluronic Acid siRNA Conjugate for Target Specific Gene Silencing", Bioconjugate Chemistry, vol. 24, no. 7, 3 June 2013 (2013-06-03), pages 1201-1209, XP055732941, ISSN: 1043-1802, DOI: 10.1021/bc4001257
- Kitae Park; Sung Woo Hong; Wonhee Hur; Min-Young Lee; Jeong-A Yang; Sung Woo Kim; Seung Kew Yoon; SeiKwang Hahn: "Target Specific Systemic Delivery of TGF- siRNA/(PEI-SS)-g-HA Complex for the Treatment of Liver Cirrhosis", Biomaterials, vol. 32, no. 21, 31 July 2011 (2011-07-31) , pages 4951-4958, XP028209390, ISSN: 0142-9612, DOI: 10.1016/j.biomaterials.2011.03.044
- PARK, Saibom et al.: "Dual Roles of Hyaluronic Acids in Multilayer Films Capturing Nanocarriers for Drug-Eluting Coatings", Biomaterials, vol. 33, no. 21, 26 April 2012 (2012-04-26), pages 5468-5477, XP028487156, ISSN: 0142-9612, DOI: 10.1016/j.biomaterials.2012.04.005

## Description

### Technical Field

The present disclosure belongs to the technical field of biochemistry, and specifically relates to a preparation method and the use of an artificial exosome complex.

### Background of the Invention

The effectiveness of a skin care product mainly depends on the absorption efficiency of an active substance, but there are few effective media for delivering active substances on the present market. Linear polyethyleneimine (PEI) has both hydrophilic and lipophilic groups, such that the linear PEI can form a bilayer with the hydrophilic groups inside and the lipophilic groups outside, and can encapsulate a substance to form a microcapsule. The microcapsule can fuse with a cell membrane due to its own membrane fusion property and then deliver an inclusion into a cell. However, due to the utilization of the membrane fusion property, the microcapsule has no cell selectivity. Hyaluronic acid (HA), also known as hyaluronan, is a natural moisturizing lubricant having a large molecular weight, and HA cannot be effectively absorbed by deep skin cells, which exhibits limited skin care persistence.

There is no combination of linear PEI and HA on the present market. A technical difficulty for the combination of linear PEI and HA is that linear PEI and HA are not simply mixed together, but are truly linked together through an amidation reaction to form a microcapsule similar to an exosome. Linear PEI and HA are both high polymers, rather than small-molecule compounds, so there is an extremely high technical difficulty in the control of such a reaction and a product quality.

CN 102 408 498 A discloses a carrier comprising as raw material PEI (MW 25,000Da) and HA (132,000 Da) but it does not disclose the use of linear PEI. It fails to describe the administration to skin.

Lutz Tauhardt et al (Lutz Tauhardt et al: "Linear Polyethyleneimine: Optimized Synthesis and Characterization - On the Way to “Pharmagrade” Batches", Macromolecular Chemistry and Physics, vol. 212, no. 17, 30 June 2011 (2011-06-30), pages 1918-1924) and US 2010/197888 A1 disclose the synthesis of linear PEI from poly(2-ethyl-2- oxazoline. They do not disclose a carrier comprising linear PEI and HA nor a carrier capable of being administered to the skin.
Kitae Park et al. discloses a carrier complex comprising HA (100 kDa) and a linear PEI (25 kDa) with lower toxicity. The carrier is used as a delivery system for siRNA suitable for tail vein injection. It is silent about transdermal administration. (Kitae Park et al,: "Reducible Hyaluronic Acid-siRNA Conjugate for Target Specific Gene Silencing", Bioconjugate Chemistry, vol. 24, no. 7, 3 June 2013 (2013-06-03), pages 1201-1209)

US 2012/202283 A1 discloses a carrier complex comprising HA and linear PEI (25kDa). The carrier is used for local administration (intratumoral) or systemic administration (intravenous) of a DNA complex. It is silent about transdermal administration.

WO 2016/144766 A1 discloses a carrier complex comprising HA, linear PEI and poly-metformin for IV administration. It is silent about transdermal administration.
Saibom Park et al, discloses a carrier complex comprising HA (1630 kDa) and linear PEI (25 kDa) as a promising drug eluting system to prevent restenosis. It is silent about transdermal administration. (SAIBOM PARK ET AL: "Dual roles of hyaluronic acids in multilayer films capturing nanocarriers for drug-eluting coatings", BIOMATERIALS, ELSEVIER, AMSTERDAM, NL, vol. 33, no. 21, 26April 2012 (2012-04-26), pages 5468-5477)
Suniti Misra et al, discloses using HA as a carrier for transdermal administration, but it does not disclose a carrier comprising linear PEI and HA for promoting the skin absorption. (Suniti Misra et al,: "Interactions between Hyaluronan and Its Receptors (CD44, RHAMM) Regulate the Activities of Inflammation and Cancer", Frontiers in Immunology, vol. 6, 6 May 2015 (2015-05-06), pages 1-31)

### Summary of the Invention

At least one technical problem solved by the present disclosure is to provide a preparation method and the use of a new artificial exosome complex. The present disclosure is intended to fuse linear PEI and HA, such that HA and PEI can play a synergistic role in a skin care product through the ligand activity of HA and the membrane permeability of PEI to facilitate the effective absorption of an active substance into deep skin cells.

In order to achieve the above objective, the present disclosure adopts the following technical solutions: An artificial exosome complex for use in promoting the absorption of an active substance in a deep skin layer through an HA receptor is provided, where raw materials for preparing the artificial exosome complex include linear PEI and HA.

Further, the HA may be composed of disaccharide units that each are formed by D-glucuronic acid and N-acetylglucosamine, and the HA may have a molecular weight of 400 to 1,800,000 and may be in the form of HA or sodium hyaluronate (SH).

Further, the linear PEI may be obtained by heating and hydrolyzing poly(2-ethyl-2-oxazoline) in a sulfuric acid aqueous solution, and may have a molecular weight of 1,000 to 100,000.

Further, the artificial exosome complex may further include an amido bond, or include ethyl, imino, tertiary amino, amido, etc.

Further, the artificial exosome complex may be prepared into an aqueous solution with a concentration of 1 mg/mL to 1 g/mL.

A preparation method of the artificial exosome complex is also provided, including the following steps:
a. dissolving 45 g of poly(2-ethyl-2-oxazoline) in 200 mL of a 30% sulfuric acid aqueous solution to obtain a reaction solution, and setting up a heating reflux device;
b. heating the reaction solution to allow a reaction at reflux, and continuously detecting a pH of a distilled solution to determine propionic acid (a reaction by-product) until there is no propionic acid in the distilled solution; and when the pH of the distilled solution is 6.0 to 7.0, stopping the reaction; wherein the propionic acid can form an azeotrope with water, and the reaction takes about 7 d;
c. after the reaction was stopped, cooling a resulting reaction system to 0°C, and starting clockwise one-way mechanical stirring at a speed of 300 rpm; and adjusting a pH of the reaction system to 7.0 by adding a sodium hydroxide solution with a concentration of 1 mol/L dropwise, and adjusting the pH to 10 to 11 by adding a small amount of the sodium hydroxide solution dropwise;
d. during the process of adjusting the pH, precipitating out a solid slowly, wherein the solid is PEI;
e. after the solid is completely precipitated out, filtering out the solid, and washing the solid with a large amount of distilled water until a resulting filtrate changes to neutral from alkaline;
f. vacuum-drying the solid at room temperature until there is no residual moisture, wherein the vacuum-drying takes about 7 d;
g. adding 600 mg of HA and 300 mL of distilled water to a beaker 1 to prepare a solution A; adding 12 g of the PEI and 300 ml of distilled water to a beaker 2 to prepare a solution B; adding the solution A to the solution B, and adjusting a pH of a resulting mixed solution to 6.5 with a 1 M HCI solution to obtain a solution C; dissolving 6.3 mmol of 1-ethyl-3-(3-dimethylaminopropyl)carbodiimide (EDC, water-soluble carbodiimide) in 15 mL of distilled water to obtain a first solvent, dissolving 6.3 mmol of 1-hydroxybenzotriazole (HOBt) in 15 mL of dimethyl sulfoxide (DMSO) to obtain a second solvent, and mixing the two solvents to obtain a solution D; and adding the solution C and the solution D to a 1.5 L round-bottom flask, stirring a resulting mixed solution at room temperature for 24 h, and adjusting a pH of the resulting mixed solution to 7.0 with a 1 M NaOH solution to obtain a reaction solution;
h. adding the reaction solution to an activated dialysis bag (8,000 KD to 14,000 KD), and dialyzing in a 100 mM NaCl solution for 2 d, then in ethanol with a volume fraction of 25% for 1 d, and finally in pure water for 1 d to obtain an HA-PEI liquid; and lyophilizing the HA-PEI liquid for 2 d to obtain a white spongy HA-PEI substance;
i. dispersing 10 g of the lyophilized HA-PEI into 50 mL of distilled water, adjusting a pH to 7.1 with a dilute hydrochloric acid solution, and adding distilled water to 100 mL; and
j. using a 0.2 µm filter membrane to sterilize a resulting solution to obtain an HA-PEI stock solution with a concentration of 100 mg/mL.

The use of the artificial exosome complex is also provided, where the artificial exosome complex is prepared into a preparation capable of significantly promoting the absorption of an active substance in a deep skin layer through an HA receptor.

Further, the preparation may be in any clinically-acceptable dosage form.

Further, the artificial exosome complex may be used as the sole active ingredient to prepare the preparation capable of promoting the absorption of an active substance by skin, or is used in combination with other substances to prepare the preparation capable of promoting the absorption of an active substance by skin.

Compared with the prior art, the present disclosure has the following advantages:
1) According to the HA-PEI preparation process of the present disclosure, linear PEI can be obtained. Because most PEI is branched currently and branched PEI has few imino groups, it is difficult to form a microcapsule structure. Therefore, linear PEI is one of the key advantages to form the artificial exosome.
2) With the HA-PEI preparation method of the present disclosure, HA-PEI with a specific molecular weight can be quantitatively prepared. Because a molecular weight of HA-PEI depends on molecular weights of HA and PEI and the molecular weight of PEI depends on a molecular weight of poly(2-ethyl-2-oxazoline), HA-PEI with a specific molecular weight can be quantitatively prepared, that is, a size of the artificial exosome can be quantitatively controlled.
3) The operation steps of the present disclosure are controllable, which is suitable for large-scale industrial production. Because the preparation process of the present disclosure is safe and environmentally-friendly and has no amplification effect, the conditions for industrial production are met.
4) The HA-PEI of the present disclosure can promote the absorption of an encapsulated active substance by deep skin cells through the ligand activity of HA and the membrane permeability of linear PEI.

### Brief description of the Drawings

FIG. 1 shows the skin irritation detection result for the artificial exosome according to one aspect of the present disclosure (Control: control group; and Exocrinetrater: artificial exosome group);
FIG. 2 shows the influence of the artificial exosome on the morphology of HaCat cells according to one aspect of the present disclosure (Control: control group; and Exocrinetrater: artificial exosome group);
FIG. 3 shows the influence of the artificial exosome on the proliferation of HaCat cells according to one aspect of the present disclosure (Control: control group; and Exocrinetrater: artificial exosome group);
FIG. 4 shows the detection result of the delivery efficiency of the artificial exosome for pCDNA3.1-eGFP according to one aspect of the present disclosure;
FIG. 5 shows the detection result of the delivery route of the artificial exosome according to one aspect of the present disclosure (DAPI: nuclear stain; Exocrinetrater: artificial exosome; and Mitochondria: mitochondrion); and
FIG. 6 shows the influence of the artificial exosome on the activity of rat skin according to one aspect of the present disclosure (Control: control group; and Exocrinetrater: artificial exosome group).

### Detailed Description of Embodiments

The present disclosure is further explained below in conjunction with specific examples, but the present disclosure is not limited thereto.

In the following examples, unless otherwise specified, the methods used are conventional methods, and all reagents used are commercially-available products.

### Example 1. Detection of skin irritation of the artificial exosome complex

The detection method is as follows. 10 volunteers were recruited; a freshly-prepared artificial exosome solution with a concentration of 1 mg/mL was evenly applied at an inner side of the left wrist, and double distilled water (DDW) was applied at an inner side of the right wrist, which used as a control group; and after the application, the changes of the inner side of the wrist that was standing was continuously observed for 20 min and it was recorded whether the volunteer had pain, itch, and other irritating symptoms.

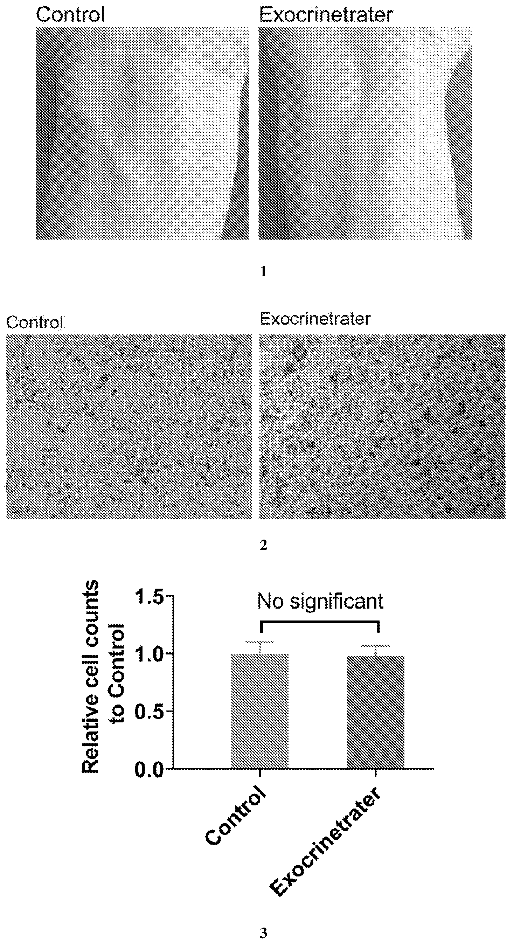

Experimental results were shown in FIG. 1.

**Table 1. Statistical results of the skin test for the artificial exosome**

| | | Control (number of volunteers) | Exocrinetrater (number of volunteers) |
|---|---|---|---|
| Pain | Yes | 0 | 0 |
| | No | 10 | 10 |
| Itch | Yes | 0 | 0 |
| | No | 10 | 10 |
| Redness and Swelling | Yes | 0 | 0 |
| | No | 10 | 10 |

The experimental conclusion is as follows.

Within the 20 min after the artificial exosome was applied, none of the 10 volunteers experienced the skin irritation symptom of redness and swelling, and none of the 10 volunteers had irritating pain, itch, etc.

### Example 2. Detection of cytotoxicity of the artificial exosome

The detection method is as follows.

Human immortal keratinocyte cells (HaCaT cells) were cultivated, digested, and inoculated in a 96-well plate at 5,000 cells/well; the artificial exosome was prepared into artificial exosome solutions with gradient concentrations, the solutions were added to the HaCaT cells, separately, and the cells were further cultivated for 48 h, the morphology of the cells was observed during the 48 h; and finally the MTT method was used to detect the influence of the artificial exosome on cell proliferation.

Experimental results were shown in FIG. 2 and FIG. 3.

The experimental conclusion is as follows.

The artificial exosome did not show obvious cytotoxicity at the cell level, and had no influence on the cell proliferation.

### Example 3. Detection of the delivery efficiency of the artificial exosome for an active substance

The detection method is as follows.

The activity of the green fluorescent protein (GFP)-encoding plasmid pCDNA3.1-eGFP that was delivered by the artificial exosome into HaCat cells was detected.

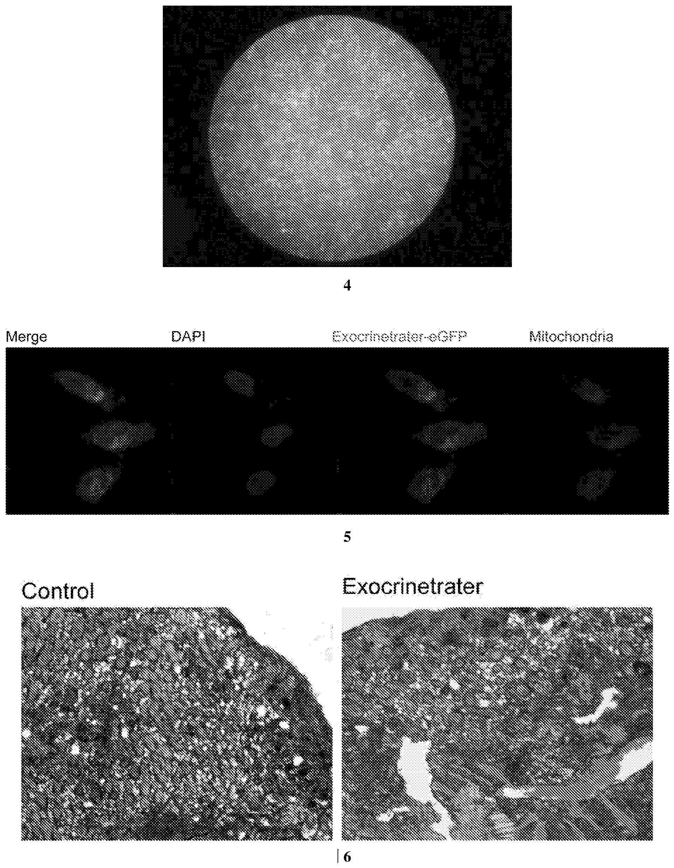

Experimental results were shown in FIG. 4.

The experimental conclusion is as follows.

The artificial exosome can well encapsulate an active substance and effectively deliver the active substance into cells.

### Example 4. Detection of the delivery route of the artificial exosome for an active substance

The detection method is as follows.

In order to further detect the delivery route of the artificial exosome for an active substance, intracellular organelles such as mitochondrion and lysosome were stained, and at the same time, the artificial exosome was used to deliver the GFP-encoding plasmid pCDNA3.1-eGFP. The delivery area of the active substance was analyzed through the positioning information of fluorescence.

Experimental results were shown in FIG. 5.

The experimental conclusion is as follows.

It shows that the artificial exosome can directly deliver the active substance to mitochondria, and it is speculated that, through this delivery route, the active substance can regulate the mitochondrial function, promote the stability of the mitochondrial function, and finally achieve the effect of anti-inflammation and cytothesis.

### Example 5. Detection of the influence of the artificial exosome on the activity of rat skin

The detection method is as follows.

Vista rats were used as experimental animals, and sodium carboxymethyl cellulose (CMC-Na) was used to prepare an artificial exosome encapsulating an active substance into an artificial exosome solution with a concentration of 1 mg/mL. The artificial exosome solution was continuously applied on the back of the rats for one week, and the control group was applied with a CMC-Na gel in which the active substance was dissolved. One week later, skin tissue was collected from the back of rats, immunofluorescence assay (IFA) was conducted to detect the expression of the active protein Cytokeratin for epidermal cells, and the functional penetration of the artificial exosome was reflected through the intensity of fluorescence.

Experimental results were shown in FIG. 6.

The experimental conclusion is as follows.

Epithelial cells, when in an active state, express the membrane protein. In the IFA, the expression of Cytokeratin can be reflected through the intensity of green fluorescence, thereby reflecting the activity of epidermal cells. It can be seen from the results that the active substance without the artificial exosome cannot effectively stimulate the expression of Cytokeratin, that is, the cell activity cannot be effectively activated; in contrast, the active substance in the artificial exosome stimulates the high expression of Cytokeratin on the skin tissue cells, that is, the artificial exosome activates the activity of epidermal cells and is conducive to the absorption of an active substance by cells. Moreover, the experimental results show that the artificial exosome can effectively activate the activity of deep skin cells. Since the artificial exosome includes HA, it is speculated that cells in the superficial skin layer such as the cuticular layer and stratum lucidum cannot effectively adsorb the artificial exosome as it cannot effectively express an HA receptor, such that the active substance can be delivered deeply to the prickle cell layer and basal cell layer with HA receptors, which makes the skin care more effective.

Although the examples of the present disclosure have been illustrated and described, it should be understood that those of ordinary skill in the art may make various changes, modifications, replacements and variations to the above examples without departing from the principle and spirit of the present disclosure, and the scope of the present disclosure is limited by the appended claims and legal equivalents thereof.

## Claims

1. An artificial exosome complex for use in promoting the absorption of an active substance in a deep skin layer through an HA receptor, wherein raw materials for preparing the artificial exosome complex comprise linear polyethyleneimine (PEI) and hyaluronic acid (HA).

2. The artificial exosome complex for use in according to claim 1, wherein the HA disaccharide units composed of D-glucuronic acid and N-acetylglucosamine, and the HA has a molecular weight of 400 to 1,800,000 and is in the form of HA or sodium hyaluronate (SH).

3. The artificial exosome complex for use in according to claim 1, wherein the linear PEI is obtained by heating and hydrolyzing poly(2-ethyl-2-oxazoline) in a sulfuric acid aqueous solution, and has a molecular weight of 1,000 to 100,000.

4. A preparation method of the artificial exosome complex for use in according to claim 1, comprising the following steps:
a. dissolving 45 g of poly(2-ethyl-2-oxazoline) in 200 mL of a 30% sulfuric acid aqueous solution to obtain a reaction solution, and setting up a heating reflux device;
b. heating the reaction solution to allow a reaction at reflux, and continuously detecting a pH of a distilled solution to determine propionic acid (a reaction by-product) until there is no propionic acid in the distilled solution; and when the pH of the distilled solution is 6.0 to 7.0, stopping the reaction; wherein the propionic acid can form an azeotrope with water, and the reaction takes about 7 d;
c. after the reaction was stopped, cooling a resulting reaction system to 0°C, and starting clockwise one-way mechanical stirring at a speed of 300 rpm; and adjusting a pH of the reaction system to 7.0 by adding a sodium hydroxide solution with a concentration of 1 mol/L dropwise, and adjusting the pH to 10 to 11 by adding a small amount of the sodium hydroxide solution dropwise;
d. during the process of adjusting the pH, precipitating out a solid slowly, wherein the solid is PEI;
e. after the solid is completely precipitated out, filtering out the solid, and washing the solid with a large amount of distilled water until a resulting filtrate changes to neutral from alkaline;
f. vacuum-drying the solid at room temperature until there is no residual moisture, wherein the vacuum-drying takes about 7 d;
g. adding 600 mg of HA and 300 mL of distilled water to a beaker 1 to prepare a solution A; adding 12 g of the PEI and 300 ml of distilled water to a beaker 2 to prepare a solution B; adding the solution A to the solution B, and adjusting a pH of a resulting mixed solution to 6.5 with a 1 M HCI solution to obtain a solution C; dissolving 6.3 mmol of 1-ethyl-3-(3-dimethylaminopropyl)carbodiimide (EDC, water-soluble carbodiimide) in 15 mL of distilled water to obtain a first solvent, dissolving 6.3 mmol of 1-hydroxybenzotriazole (HOBt) in 15 mL of dimethyl sulfoxide (DMSO) to obtain a second solvent, and mixing the first solvent and the second solvent to obtain a solution D; and adding the solution C and the solution D to a 1.5 L round-bottom flask, stirring a resulting mixed solution at room temperature for 24 h, and adjusting a pH of the resulting mixed solution to 7.0 with a 1 M NaOH solution to obtain a reaction solution;
h. adding the reaction solution to an activated dialysis bag of 8,000 KD to 14,000 KD, and dialyzing in a 100 mM NaCl solution for 2 d, then in ethanol with a volume fraction of 25% for 1 d, and finally in pure water for 1 d to obtain an HA-PEI liquid; and lyophilizing the HA-PEI liquid for 2 d to obtain a white spongy HA-PEI substance;
i. dispersing 10 g of the lyophilized HA-PEI into 50 mL of distilled water, adjusting a pH to 7.1 with a dilute hydrochloric acid solution, and adding distilled water to 100 mL; and
j. using a 0.2 µm filter membrane to sterilize a resulting solution to obtain an HA-PEI stock solution with a concentration of 100 mg/mL.

5. The artificial exosome complex for use in according to claim 1, wherein the artificial exosome complex further comprises an amido bond, or comprises ethyl, imino, tertiary amino, amido, etc.

6. The artificial exosome complex for use in according to claim 1, wherein the artificial exosome complex is prepared into an aqueous solution with a concentration of 1 mg/mL to 1 g/mL.

7. A clinically-acceptable dosage form comprising the artificial exosome complex for use in according to claim 1.

8. A preparation comprising the artificial exosome complex for use in according to claim 1, wherein the artificial exosome complex is used as a sole active ingredient to prepare the preparation capable of promoting the absorption of the active substance by skin, or is used in combination with other substances to prepare the preparation capable of promoting the absorption of the active substance by skin.

## Patentansprüche

1. Künstlicher Exosomenkomplex zur Verwendung in der Förderung der Absorption einer Wirksubstanz in einer tiefen Hautschicht über einen HA-Rezeptor, wobei die Rohstoffe zur Herstellung des künstlichen Exosomenkomplexes lineares Polyethylenimin (PEI) und Hyaluronsäure (HA) umfassen.

2. Künstlicher Exosomenkomplex zur Verwendung gemäß Anspruch 1,
wobei die HA-Disaccharid-Einheit aus D-Glucuronsäure und N-Acetylglucosamin besteht und die HA ein Molekulargewicht von 400 bis 1.800.000 hat und als HA oder Natriumhyaluronat (SH) vorliegt.

3. Künstlicher Exosomenkomplex zur Verwendung gemäß Anspruch 1,
wobei das lineare PEI durch Erhitzen und Hydrolysieren von Poly(2-ethyl-2-oxazolin) in einer wässrigen Schwefelsäurelösung erhalten wird und ein Molekulargewicht von 1.000 bis 100.000 hat.

4. Herstellungsverfahren für den künstlichen Exosomenkomplex zur Verwendung gemäß Anspruch 1, umfassend
die folgenden Schritte:
a. Auflösen von 45 g Poly(2-ethyl-2-oxazolin) in 200 mL einer zu 30% wässrigen Schwefelsäurelösung, um eine Reaktionslösung zu erhalten, und Aufstellen einer Rückflussheizvorrichtung;
b. Erhitzen der Reaktionslösung, um eine Rückflussreaktion zu ermöglichen, und laufende Bestimmen des pH-Werts einer destillierten Lösung, um Propionsäure (ein Reaktionsnebenprodukt) zu ermitteln, bis keine Propionsäure mehr in der destillierten Lösung vorhanden ist; und wenn der pH-Wert der destillierten Lösung bei 6.0 bis 7.0 liegt, Stoppen der Reaktion; wobei die Propionsäure ein Azeotrop mit Wasser bilden kann und die Reaktion etwa 7 Tage dauert;
c. nach dem Stoppen der Reaktion, Abkühlen des entstandenen Reaktionssystems auf 0°C und Beginn des mechanischen Einweg-Rührens im Uhrzeigersinn mit einer Geschwindigkeit von 300 U/min; und Einstellen eines pH-Wertes des Reaktionssystems auf 7.0, indem eine Natriumhydroxidlösung mit einer Konzentration von 1 mol/L tropfenweise zugegeben wird, und Einstellen des pH-Wertes auf 10 bis 11, indem eine kleine Menge der Natriumhydroxidlösung tropfenweise zugegeben wird;
d. während des Einstellungsprozesses des pH-Wertes, langsames Ausfällen eines Feststoffes, wobei der Feststoff PEI ist;
e. nachdem der Feststoff vollständig ausgefallen ist, Filtern des Feststoffs und Waschen des Feststoffs mit einer großen Menge destillierten Wassers, bis das entstandene Filtrat nicht mehr alkalisch, sondern neutral ist,
f. Vakuumtrocknen des Feststoffs bei Raumtemperatur, bis keine verbleibende Feuchtigkeit mehr vorhanden ist, wobei die Vakuumtrocknungsdauer etwa 7 Tage beträgt;
g. Zugabe von 600 mg HA und 300 mL destilliertem Wasser zu einem Becherglas (1) zur Herstellung einer Lösung A; Zugabe von 12 g PEI und 300 ml destilliertem Wasser zu einem Becherglas (2) zur Herstellung einer Lösung B; Zugabe der Lösung A zu der Lösung B und Einstellen des pH-Werts der entstandenen Mischlösung auf 6.5 mit einer 1 M HCl-Lösung, um eine Lösung C zu erhalten, Auflösen von 6.3 mmol 1-Ethyl-3-(3-dimethylaminopropyl)Carbodiimid (EDC, wasserlösliches Carbodiimid) auf 15 mL destilliertes Wasser, um ein erstes Lösungsmittel zu erhalten, Auflösen von 6.3 mmol 1-Hydroxybenzotriazol (HOBt) auf 15 mL Dimethylsulfoxid (DMSO), um ein zweites Lösungsmittel zu erhalten, und Mischen des ersten Lösungsmittels und des zweiten Lösungsmittels, um eine Lösung D zu erhalten; und Zugabe der Lösung C und der Lösung D zu einem Rundkolben von 1.5 L, Rühren einer erhaltenen Mischlösung bei Raumtemperatur für 24 Stunden und Einstellen eines pH-Wertes der erhaltenen Mischlösung auf 7.0 mit einer 1 M NaOH-Lösung, um eine Reaktionslösung zu erhalten;
h. Zugabe der Reaktionslösung zu einem aktivierten Dialysebeutel von 8.000 KD bis 14.000 KD und Dialyse in einer 100 mM NaCl-Lösung für 2 Tage, danach in Ethanol mit einem Volumenanteil von 25% für 1 Tag, und abschließend in reinem Wasser für 1 Tag, um eine HA-PEI Flüssigkeit zu erhalten; und Lyophilisieren der HA-PEI-Flüssigkeit für 2 Tage, um eine weiße, schwammige HA-PEI-Substanz zu erhalten;
i. Dispergieren von 10 g des lyophilisierten HA-PEI in 50 mL destilliertem Wasser, Einstellen des pH-Werts auf 7.1 mit einer verwässerten Salzsäurelösung und Zugabe von destilliertem Wasser auf 100 mL; und
j. Sterilisieren der erhaltenen Lösung unter Verwendung einer 0.2 µm Filtermembran, um eine HA-PEI-Stammlösung mit einer Konzentration von 100 mg/ml zu erhalten.

5. Künstlicher Exosomenkomplex zur Verwendung gemäß Anspruch 1,
wobei der künstliche Exosomenkomplex ferner eine Amidobindung oder eine Etyl, Imino, tertiäre Amino, Amido, usw. umfasst.

6. Künstlicher Exosomenkomplex zur Verwendung gemäß Anspruch 1,
wobei der künstliche Exosomenkomplex in einer wässrigen Lösung mit einer Konzentration von 1 mg/ml bis 1 g/ml hergestellt wird.

7. Klinisch akzeptable Dosierungsform, umfassend den künstlichen Exosomenkomplex zur Verwendung gemäß Anspruch 1.

8. Präparation, umfassend den künstlichen Exosomenkomplex zur Verwendung gemäß Anspruch 1,
wobei der künstliche Exosomenkomplex als einziger Wirkstoff zur Herstellung der Präparation verwendet wird, die geeignet ist, die Absorption der Wirksubstanz durch die Haut zu fördern, oder in Kombination mit anderen Substanzen zur Herstellung der Präparation verwendet wird, die geeignet ist, die Absorption der Wirksubstanz durch die Haut zu fördern.

## Revendications

1. Complexe d'exosome artificiel pour l'utilisation dans la facilitation de l'absorption d'une substance active dans une couche cutanée profonde par l'intermédiaire d'un récepteur HA, dans lequel des matières premières pour la préparation du complexe d'exosome artificiel comprennent la polyéthylèneimine linéaire (PEI) et l'acide hyaluronique (HA).

2. Complexe d'exosome artificiel pour l'utilisation selon la revendication 1, dans lequel une unité de disaccharide HA est composée d'acide D-glucuronique et de N-acétylglucosamine, et l'HA présente un poids moléculaire de 400 à 1 800 000 et se présente sous forme d'HA ou d'hyaluronate de sodium (SH).

3. Complexe d'exosome artificiel pour l'utilisation selon la revendication 1, dans lequel le PEI linéaire est obtenu en chauffant et hydrolysant poly(2-éthyl-2-oxazoline) dans une solution aqueuse d'acide sulfurique, et présente un poids moléculaire de 1 000 à 100 000.

4. Procédé de préparation du complexe d'exosome artificiel pour l'utilisation selon la revendication 1, comprenant les étapes suivantes :
a. dissoudre 45 g de poly(2-éthyl-2-oxazoline) dans 200 mL de solution aqueuse d'acide sulfurique à 30 % pour obtenir une solution réactionnelle, et réguler un dispositif de reflux chauffant ;
b. chauffer la solution réactionnelle pour permettre une réaction au reflux, et détecter en continu le pH d'une solution distillée pour déterminer l'acide propionique (un sous-produit de la réaction) jusqu'à ce qu'il n'y ait plus d'acide propionique dans la solution distillée ; et arrêter la réaction lorsque le pH de la solution distillée est de 6,0 à 7,0 ; dans lequel l'acide propionique peut former un azéotrope avec de l'eau, et la réaction dure environ 7 jours ;
c. une fois la réaction arrêtée, refroidir le système de réaction résultant à 0 °C et démarrer une agitation mécanique unidirectionnelle dans le sens des aiguilles d'une montre à une vitesse de 300 tr/min ; et ajuster le pH du système réactionnel à 7,0 en ajoutant goutte à goutte une solution d'hydroxyde de sodium à une concentration de 1 mol/L, et ajuster le pH à 10 à 11 en ajoutant goutte à goutte une petite quantité de solution d'hydroxyde de sodium ;
d. lors de l'ajustement du pH, précipiter doucement une phase solide, dans lequel la phase solide est PEI ;
e. une fois que la phase solide est complètement précipitée, filtrer la phase solide et laver celle-ci avec un grand volume d'eau distillée jusqu'à ce que le filtrat résultant passe d'alcalin à neutre ;
f. sécher sous vide la phase solide à température ambiante jusqu' à ce qu'il n'y ait plus d'humidité résiduelle, dans lequel le séchage sous vide dure environ 7 jours ;
g. ajouter 600 mg d'HA et 300 mL d'eau distillée dans un becher 1 pour préparer une solution A ; ajouter 12 g de PEI et 300 ml d'eau distillée dans un becher 2 pour préparer une solution B ; ajouter la solution A à la solution B et ajuster le pH d'une solution mélangée résultante à 6,5 avec une solution HCI de 1 M pour obtenir une solution C ; dissoudre 6,3 mmol de 1-éthyl-3-(3-diméthylaminopropyl)carbodiimide (EDC, carbodiimide soluble dans l'eau) dans 15 mL d'eau distillée pour obtenir un premier solvant, dissoudre 6,3 mmol de 1-hydroxybenzotriazole (HOBt) dans 15 mL de diméthylsulfoxyde (DMSO) pour obtenir un deuxième solvant, et mélanger le premier solvant et le deuxième solvant pour obtenir une solution D ; et ajouter la solution C et la solution D dans une fiole à fond rond de 1,5 L, agiter une solution mélangée obtenue à température ambiante pendant 24 h et ajuster le pH de la solution mélangée résultante à 7,0 avec une solution de NaOH de 1 M pour obtenir une solution réactionnelle ;
h. ajouter la solution réactionnelle à une poche de dialyse activée de 8 000 KD à 14 000 KD, et dialyser dans une solution de NaCl à 100 mM pendant 2 jours, puis dans l'éthanol à une fraction volumique de 25 % pendant 1 jour, et enfin dans l'eau pure pendant 1 jour pour obtenir un liquide HA-PEI ; et lyophiliser le liquide HA-PEI pendant 2 jours pour obtenir une substance HA-PEI blanche et spongieuse ;
i. disperser 10 g d'HA-PEI lyophilisé dans 50 mL d'eau distillée, ajuster le pH à 7,1 avec une solution diluée d'acide chlorhydrique et ajouter de l'eau distillée à 100 mL ; et
j. stériliser, à l'aide d'une membrane filtrante de 0,2 µm, une solution résultante afin d'obtenir une solution mère de HA-PEI à une concentration de 100 mg/mL.

5. Complexe d'exosome artificiel pour l'utilisation selon la revendication 1, dans lequel le complexe d'exosome artificiel comprend en outre une liaison amido, ou comprend l'éthyle, l'imino, l'amino tertiaire, l'amido, etc.

6. Complexe d'exosome artificiel pour l'utilisation selon la revendication 1, dans lequel le complexe d'exosome artificiel est préparé dans une solution aqueuse à une concentration de 1 mg/mL à 1 g/mL.

7. Forme posologique cliniquement acceptable comprenant le complexe d'exosome artificiel pour l'utilisation selon la revendication 1.

8. Préparation comprenant le complexe d'exosome artificiel pour l'utilisation selon la revendication 1, dans laquelle le complexe d'exosome artificiel est utilisé comme seul ingrédient actif pour préparer la préparation capable de faciliter l'absorption de la substance active par la peau, ou est utilisé en combinaison avec d'autres substances pour préparer la préparation capable de faciliter l'absorption de la substance active par la peau.
